Europäisches Patentamt

**European Patent Office**

**Office européen des brevets**

(11) Publication number:

**0 352 060**
**A2**

(12)

**EUROPEAN PATENT APPLICATION**

(21) Application number: **89307250.4**

(22) Date of filing: **18.07.89**

(51) Int. Cl.⁵: **C 12 N 15/81**
**C 12 N 1/18, C 12 P 21/02**
**//(C12N15/81,15:48)**

(30) Priority: **21.07.88 JP 182187/88**

(43) Date of publication of application:
**24.01.90 Bulletin 90/04**

(84) Designated Contracting States:
**AT BE CH DE ES FR GB GR IT LI LU NL SE**

(71) Applicant: **TAKEDA CHEMICAL INDUSTRIES, LTD.**
**3-6, Doshomachi 2-chome Chuo-ku**
**Osaka 541 (JP)**

(72) Inventor: **Fujisawa, Yukio**
**31-104, 1 Mikagenakamachi 4-chome Higashinada-ku**
**Kobe Hyogo 658 (JP)**

**Kato, Koichi**
**4-9, Miyamadai 2-chome .**
**Kawanishi Hyogo 666-01 (JP)**

**Hatanaka, Masakazu**
**1-23, Takanohigashihiraki-cho Sakyo-ku**
**Kyoto 606 (JP)**

(74) Representative: **Lewin, John Harvey et al**
**ELKINGTON AND FIFE Beacon House 113 Kingsway**
**London WC2B 6PP (GB)**

The applicant has filed a statement in accordance with Rule 28 (4) EPC (issue of a sample only to an expert). Accession number(s) of the deposit(s): BP-1961, BP-1962, BP- 1947, BP-1948 and BP-1059.

(54) **Expression plasmid and use thereof.**

㊗ Disclosed are an expression plasmid for a protein possessing HTLV-I gag antigenicity, a yeast transformed with said plasmid, and a method of producing a protein possessing HTLV-I gag antigenicity by means of said yeast transformant. The gag polypeptide obtained by the present invention possesses the same antigenicity as that of the gag protein produced using HTLV-I-infected cells as the starting material, thus serving as a diagnostic kit material and HTLV-I vaccine.

EP 0 352 060 A2

Description

# EXPRESSION PLASMID AND USE THEREOF

## BACKGROUND OF THE INVENTION

The present invention relates to a recombinant DNA for production of a protein possessing human T cell leukemia virus type I (hereinafter also referred to as HTLV-I) gag antigenicity. Specifically, the present invention relates to an expression plasmid for a protein possessing HTLV-I gag antigenicity, a yeast transformed with said plasmid, and a method of producing a protein possessing HTLV-I gag antigenicity by means of said yeast transformant.

## PRIOR ART

HTLV-I is the human retrovirus isolated as the causative virus of adult T cell leukemia (ATL) [T. Uchiyama et al., Blood, 50, 481 (1977)][B. Poiesz et al., Proc. Natl. Acad. Sci. USA, 77, 6815 (1980); Y. Hinuma et al., Proc. Natl. Acad. Sci. USA, 78, 6476 (1981)]. Referring to the genes of this virus, it is known that pX gene exists as well as gag (abbreviation for group specific antigen), pol (abbreviation for polymerase) and env (abbreviation for envelope) genes, the latter three all found in ordinary retroviruses [M. Yoshida, Gann, 74, 777 (1983); M. Seiki et al., Proc. Natl. Acad. Sci. USA, 80, 3618 (1983)].

Analysis of the genome structure has determined the derivation of the HTLV-I antigen molecule as follows: The gag gene encodes the inner core antigen precursor protein p53, which is processed eventually to three different core antigens, namely p19, p24 and p15. The envelope precursor protein p46, encoded by the env gene, converts itself to the precursor glycoprotein gp62 via sugar chain addition, which is processed eventually to two kinds of envelope glycoproteins, namely gp46 and gp21. In the pX gene, four open reading frames exist, pX-III yields p27 and p21, and pX-IV yields p40.

On the other hand, it is known that antibodies against the above-mentioned viral antigens [ATL-related antigens (ATLA)] are present in sera of humans infected with HTLV-I and ATL patients [Y. HINUMA et al., Proc. Natl. Acad. Sci. USA, 78, 6476 (1981)], and it is possible to use ATLA to detect antibodies against HTLV-I. Antigens utilized to detect these antibodies are HTLV-I viral antigens isolated and purified from HTLV-I producer cells (Japanese Patent Unexamined Publication No. 187861/1983), the 43-kDal LacZ'-gag fused protein [S. Itamura et al., Gene, 38, 57 (1985)] and the 100-kDal gag-env-pX-IV gene product (Japanese Patent Unexamined Publication No. 296889/1987 which corresponds to GB-A-2122343), both prepared by gene recombination technology. However, the amount of antigens obtained by these methods is very small when the method is based on cell cultivation or bacterial gene recombination (e.g. the amount of the 43-kDal LacZ'-gag fused protein produced using an Escherichia coli recombinant is 0.3%, relative to the total soluble protein); gene expression level is low.

As stated above, it has long been desired that a technique will be developed which permits mass production of a protein possessing HTLV-I gag antigenicity.

## SUMMARY OF THE INVENTION

With the aim of mass-producing a protein possessing HTLV-I gag antigenicity by gene recombination technology, the present inventors conducted intensive investigations, and succeeded in mass-producing a protein possessing gag antigenicity (hereinafter also referred to as gag polypeptide) by (1) preparing a gag gene or part thereof from a plasmid cloned with HTLV-I gag gene, (2) inserting the gag gene into an expression vector for yeasts to yield an expression plasmid for a protein possessing gag antigenicity, (3) transforming a yeast with the plasmid, and (4) cultivating the transformant to produce and accumulate in the culture broth a protein possessing gag antigenicity or its derivative, which is then harvested. The present inventors conducted further relevant investigations, and developed the present invention.

Accordingly, the present invention comprises:

(1) an expression plasmid for a protein possessing human T cell leukemia virus type I gag antigenicity, characterized in that it contains a DNA that encodes said protein and is capable of replication in yeasts,

(2) a yeast transformant transformed with the plasmid of item (1) above, and

(3) a method of producing a protein possessing human T cell leukemia virus type I gag antigenicity, characterized in that the yeast transformant of item (2) above is cultivated in a culture medium to produce and accumulate in the culture broth said protein, which is then harvested.

## BRIEF DESCRIPTION OF THE DRAWINGS

Fig. 1 shows the construction schematic of the plasmid pGLD 907T, used in Example 3.

Fig. 2 shows the method of preparing the gag gene (Xhol fragment), described in Example 1.

Fig. 3 shows the DNA base sequence of the gag gene and the amino acid sequence of the gag protein (*** represents the translation termination codon).

Fig. 4 shows the construction schematic of the expression plasmid pHTI 501, obtained in Example 2.

Fig. 5 shows the construction schematic of the expression plasmid pHTI 511, obtained in Example 3.

Fig. 6 shows the SDS-PAGE pattern of the HTLV-I gag precursor obtained in Example 5(Fig. 6A shows the SDS-PAGE pattern obtained when the silver staining method was used for staining; Fig. 6B shows the SDS PAGE pattern obtained when the Western blotting method was used for staining).

## DETAILED DESCRIPTION OF THE INVENTION

Any protein possessing gag antigenicity can be used for the present invention, as long as it reacts with antibodies against at least one of the above-mentioned three kinds of core antigens, namely p19, p24 and p15. Examples of such proteins include gag protein (gag precursor) and its derivatives possessing gag antigenicity (e.g. p19, p24, p15).

The HTLV-I gag gene or part thereof to be used for the present invention can be prepared from an HTLV-I provirus [S. H. Nam et al., Biochem. Biophys. Res. Commun., 139, 129 (1986)] using appropriate restriction enzymes; it is possible to prepare a large amount of gag gene or part thereof by amplifying in Escherichia coli a plasmid subcloned with the HTLV-I gag gene.

Any expression vector (e.g. plasmid) can be used for the present invention, as long as it is capable of replication in yeasts. Examples of the vector include pSH 19 [S. Harashima et al., Mol. Cell. Biol., 4, 771 (1984)] and pSH 19-1 (European Patent Publication No. EP-A-0235430). A vehicle for exogenous gene expression can be obtained by inserting a promoter into these vectors.

Any promoter can be used for the expression, as long as it is capable of functioning in yeasts. Examples of preferable promoters include the GLD (GAPDH) promoter, PHO 5 promoter, PGK promoter, ADH promoter, PHO 81 promoter and GAL 10 promoter.

The desired promoter can be enzymatically prepared from respective genes. Chemical synthesis is also possible.

The expression plasmid for a DNA that encodes gag polypeptide can be obtained by inserting a DNA that encodes gag polypeptide downstream of the promoter of the above-mentioned expression vector or by inserting a DNA ligated with another DNA that encodes all or part of a signal peptide that functions in yeasts into the 5'-terminal side of a DNA that encodes gag polypeptide. Examples of the expression vector include pPHO 17, pGLD 906 and pGLD 906-1 [Y. Itoh et al., Biochem. Biophys. Res. Commun., 138, 268 (1986)]. The expression efficiency for the DNA that encodes gag polypeptide can be increased by inserting a terminator downstream of the DNA encoding the gag polypeptide. Examples of the terminator include the phosphoglycerate kinase gene (PGK) terminator, 2μ DNA FLP gene terminator and invertase gene (SUC 2) terminator.

The method of constructing the expression plasmid for the present invention is already known, described in published reports such as "Molecular Cloning," Cold Spring Harbor Laboratory (1982).

Yeasts can be used as hosts to be transformed with the expression plasmid. Among them, Saccharomyces cerevisiae is preferred. Particularly, respiratory deficient strains ( ρ⁻) of S. cerevisiae are suitable as hosts.

A method of obtaining a respiratory deficient strain (ρ⁻) from a yeast capable of respiration (parent strain ρ⁺) is described in "Laboratory Course Manual for Methods in Yeast Genetics," Cold Spring Harbor Laboratory (1986), for instance. Accordingly, a respiratory deficient strain can easily be obtained by isolating a strain capable of growth in culture medium containing glucose as the carbon source but not capable of growth in culture medium containing glycerol in place of glucose, after cultivation of the parent strain in a culture medium containing ethidium bromide. It is also possible to obtain a respiratory deficient strain out of a single colony of the parent strain, though the mutation rate is low. When the parent strain is a transformant (recombinant) carrying an expression plasmid, the desired recombinant with highly efficient gene expression can be obtained directly by isolating a respiratory deficient strain of the transformant. When the parent strain does not carry an expression plasmid, the desired recombinant is obtained by deriving a respiratory deficient strain and then introducing an expression plasmid into it.

Examples of the method of transforming a yeast with a recombinant DNA (plasmid) include well-known methods such as the lithium method [H. Itoh et al., J. Bacteriol., 153, 163 (1983)] and the protoplast method [A. Hinnen et al., Proc. Natl. Acad. Sci. USA, 75, 1927 (1978)).

The transformant (recombinant) thus obtained is cultivated by known methods.

Examples of the culture medium include Burkholder minimal medium [Amer. J. Bot., 30, 206 (1943)], modified Burkholder minimal medium [A. Toh-e et al., J. Bacteriol., 113, 727 (1973)] and low phosphoric acid content medium [A. Toh-e et al., J. Bacteriol., 113, 727 (1973)]. Cultivation is normally carried out at 15 to 40°C, preferably 24 to 37°C, for 10 to 168 hours, preferably 72 to 144 hours. Shaking culture and stationary culture are both usable; aeration and/or agitation may be conducted as needed.

In accordance with the present invention, the gag polypeptide can easily be purified by a combination of known methods of protein extraction and purification such as cell disruption using glass beads, centrifugation, salt precipitation, isoelectric precipitation, gel filtration, ion exchange chromatography, high performance liquid chromatography, affinity chromatography, sucrose density gradient ultracentrifugation and cesium chloride density gradient ultracentrifugation.

The present invention allows mass production of the gag polypeptide which possesses the same antigenicity as that of the gag protein produced using HTLV-I-infected cells as the starting material, and the gag polypeptide obtained by the present invention is suitable for use in a diagnostic kit and HTLV-I vaccine.

Note that the abbreviations for bases, amino acids, etc. used in the present specification and drawings are based on abbreviations specified by the IUPAC-IUB Commission on Biochemical Nomenclature or those commonly used in relevant fields. Examples of the abbreviations are given below. When there is a possibility of the existence of an optical isomer in a particular amino acid, it is an L-enantiomer unless otherwise stated.

DNA: Deoxyribonucleic acid

RNA: Ribonucleic acid

mRNA: Messenger RNA

A: Adenine

T: Thymine

G: Guanine

C; Cytosine

dATP: Deoxyadenosine triphosphate

dTTP: Deoxythymidine triphosphate

dGTP: Deoxyguanosine triphosphate

dCTP: Deoxycytidine triphosphate

ATP: Adenosine triphosphate

EDTA: Ethylenediaminetetraacetic acid

SDS: Sodium dodecyl sulfate

DTT: Dithiothreitol

Gly: Glycine(G)

Ala: Alanine(A)

Val: Valine(V)

Leu: Leucine(L)

Ile: Isoleucine(I)

Ser: Serine(S)

Thr: Threonine(T)

Cys: Cysteine(C)

1/2Cys: Half cystine

Met: Methionine(M)

Glu: Glutamic acid (E)

Asp: Aspartic acid (D)

Lys: Lysine(K)

Arg: Arginine(R)

His: Histidine(H)

Phe: Phenylalanine(F)

Tyr: Tyrosine(Y)

Trp: Tryptophan(W)

Pro: Proline(P)

Asn: Asparagine(N)

Gln: Glutamine(Q)

$Ap^r$: Ampicillin resistance gene

$Tc^r$: Tetracycline resistance gene

ARS 1: Autonomous replication sequence 1

The accession numbers, at respective deposition organizations, of the plasmids and microorganisms used for the present invention are given below. Note that IFO represents the Institute for Fermentation, Osaka, and FRI the Fermentation Research Institute, Agency of Industrial Science and Technology, Japan.

| Microorganism | IFO (IFO No.) | FRI (FERM No.) |
|---|---|---|
| Saccharomyces cerevisiae NA74-3A( $\rho^-$ )/pHTI501 | 10440 | BP-1961 |
| Saccharomyces cerevisiae NA74-3A( $\rho^-$ )/pHTI511 | 10441 | BP-1962 |
| Saccharomyces cerevisiae NA74-3A | 10430 | BP-1947 |
| Saccharomyces cerevisiae NA74-3A( $\rho^-$ ) | 10431 | BP-1948 |
| Saccharomyces cerevisiae AH22R⁻/pGLD P31-RcT | 10206 | BP-1059 |

Saccharomyces cerevisiae NA74-3A ( $\rho^-$ )/pHTI 501,

Saccharomyces cerevisiae NA74-3A ( $\rho^-$ )/pHTI 511 and

Saccharomyces cerevisiae AH22R⁻/pGLD P31-RcT respectively

carry the plasmids pHTI 510, pHTI 511 and pGLD P31-RcT.

## EXAMPLES

The present invention is hereinafter described in more detail by means of the following reference examples and working examples; these examples are not to be construed as limitations on the invention.

Reference Example 1

Preparation of respiratory deficient strain
A respiratory deficient strain( p⁻) of Saccharomyces cerevisiae NA74-3A was isolated using ethidium bromide in accordance with the method described in "Laboratory Course Manual for Methods in Yeast Genetics", Cold Spring Harbor Laboratory(1986).

Reference Example 2

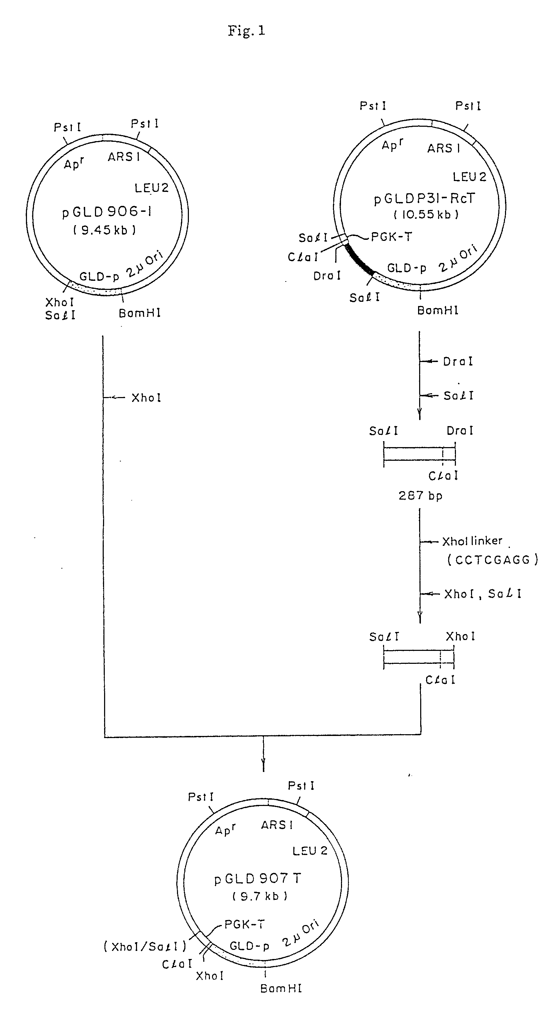

Construction of Expression Vector pGLD907T
The expression vector pGLD907T was constructed by inserting into the XhoI site of an expression vector pGLD906-1 [Itoh., Y. et al., Biochem. Biophys. Res. Commun., 138, 268(1986)] a DNA fragment which was obtained by addition of an XhoI linker d(CCTCGAGG) to a 287 bp DraI-SalI DNA fragment obtained from a plasmid pGLD P31-RcT (European Patent Publication EP-A-0235430) (Fig. 1).

Example 1

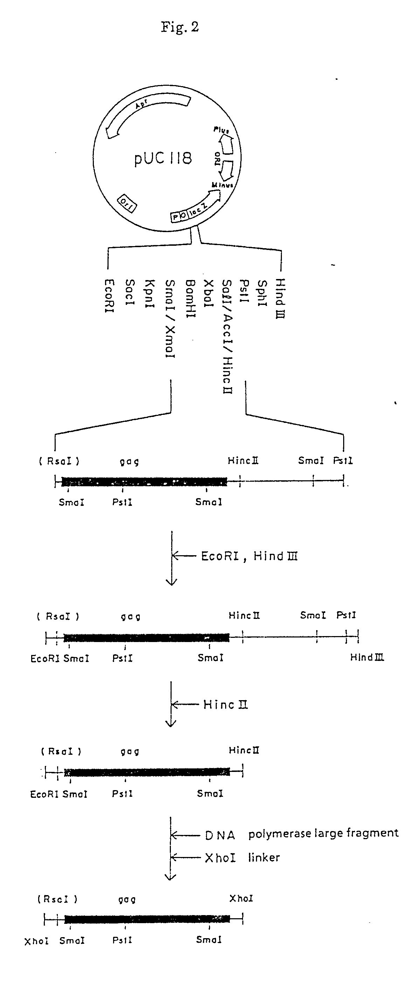

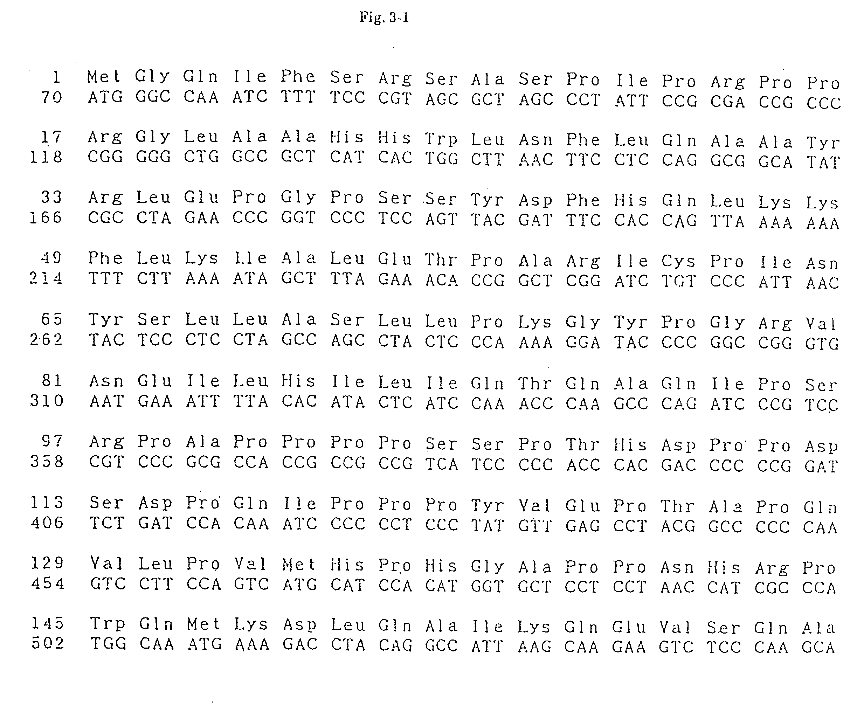

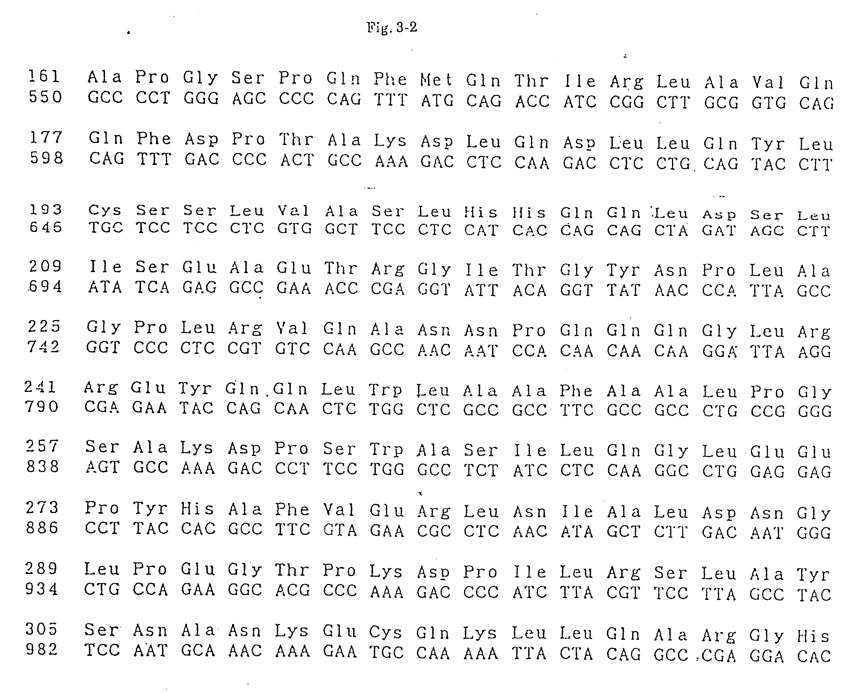

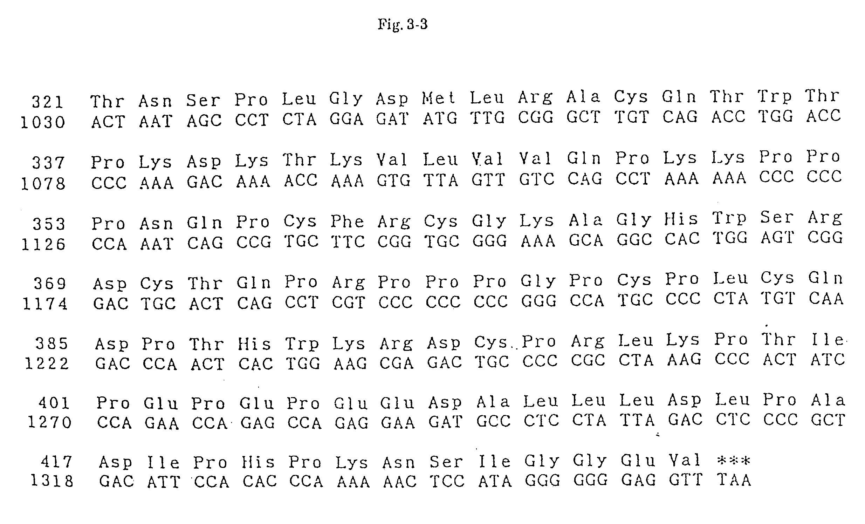

Preparation of gag Gene
A known HTLV-I provirus HTLV IC [S.H. Nam et al., Biochem. Biophys. Res. Commun., 139, 129(1986)] was partially digested with RsaI and PstI to prepare a 2.3 kb DNA fragment containing the region of 3'-end LTR(part)-gag-prt-pol(part). The fragment was inserted into pUC118 (Takara Shuzo) digested with SmaI and PstI. The recombinant DNA was digested with EcoRI and HindIII to prepare a 2.3 kb DNA fragment containing the gag gene, and then the fragment was digested with HincII to prepare a 1.5 kb DNA fragment containing 3′-end LTR(part)-gag-prt(part). The fragment was treated with DNA polymerase I large fragment (Takara Shuzo), and an XhoI linker (New England Biolabs., USA) was added to the fragment with T4 DNA ligase and then the fragment was digested with XhoI to expose XhoI sites (Fig. 2). The DNA base sequence of the gag gene contained in the fragment is shown in Fig. 3.

Example 2

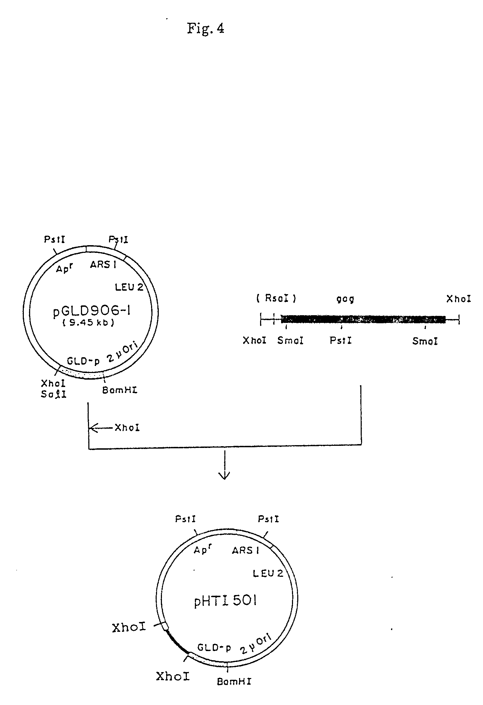

Construction of Expression Plasmid-1
The gag gene having XhoI sites at both ends which was obtained in Example 1 was inserted into a yeast expression plasmid pGLD906-1 [Y. Itoh et al., Biochem. Biophys. Res. Commun., 138, 268 (1986)] which was previously digested with XhoI to construct an expression plasmid pHTI 501 (Fig. 4).

Example 3

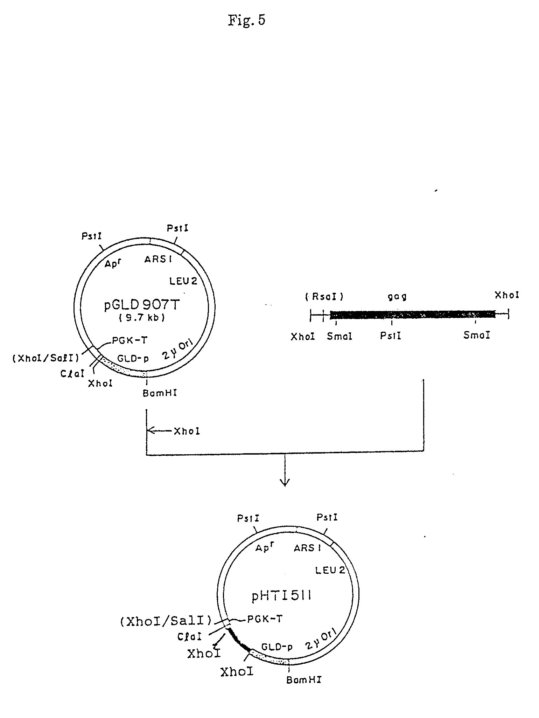

Construction of Expression Plasmid-2
The gag gene having XhoI sites at both ends which was obtained in Example 1 was inserted into a yeast expression plasmid pGLD907T (described in Reference Example 2) which was previously digested with XhoI to construct an expression plasmid pHTI 511 (Fig. 5).

Example 4

Preparation and Cultivation of Yeast Transformants and Expression of gag Protein
Yeast Saccharomyces cerevisiae NA74-3A(p⁻) was transformed with plasmids pHTI 501 and pHTI 511 obtained in Reference Examples 2 and 3 by the protoplast method [A. Hinnen et al., Proc. Natl. Acad. Sci. USA, 75, 1927 (1978)] to obtain transformants Saccharomyces cerevisiae NA74-3A (p⁻)/pHTI 501 and Saccharomyces cerevisiae NA74-3A(p⁻)/pHTI 511, respectively.
Each of these yeast transformants was cultivated at 30°C for 3 days with shaking in 5 ml of a culture medium [3g of $K_2HPO_4$, 50 g of glucose, 4 g of L-asparagine, 100 mg of L-histidine, 0.1 mg of KI, 500 mg of $MgSO_4$ ·$7H_2O$, 330 mg of $CaCl_2·2H_2O$, 0.4 mg of $CuSO_4·5H_2O$, 2.5 mg of $FeSO_4·7H_2O$, 0.4 mg of $MnSO_4·4H_2O$, 0.2mg of $(NH_4)_3PO_4·12MoO_3·3H_2O$, 3.1 mg of $ZnSO_4·7H_2O$, 10 mg of inositol, 0.2 mg of thiamine, 0.2 mg of pyridoxine, 0.2 mg of Ca-pantothenate, 0.2 mg of niacin and 0.002 mg of biotin, per liter], and 0.5 ml of the culture was transferred to 4.5 ml of another culture broth having the same composition as mentioned above and cultivated at 30°C for 1 day. Then, 2 ml of the culture was transferred to 18 ml of a fresh medium [400 mg of $KH_2PO_4$, 80 g of sucrose, 5 g of L-asparagine, 300 mg of L-histidine, 2.0 g of KCl, 0.1 mg of KI, 650 mg of $MgSO_4$ ·$7H_2O$, 429 mg of $CaCl_2·2H_2O$, 10 g of glucose, 25 mM of Tris-maleic acid (pH 6.5), 0.4 mg of $CuSO_4·5H_2O$, 2.5 mg of $FeSO_4.7H_2O$, 0.4 mg of $MnSO_4·4H_2O$, 0.2 mg of $(NH_4)_3PO_4·12MoO_3·3H_2O$, 3.1 mg of $ZnSO_4·7H_2O$, 10 mg of inositol, 0.2 mg of thiamine, 0.2 mg of pyridoxine, 4.0 mg of Ca-pantothenate, 4.0 mg of niacin and 0.040 mg of biotin, per liter] and cultivated at 30°C with shaking. After 72 hours, the culture was sampled and centrifuged (10,000 x g, 10 minutes) for separation into a supernatant and cells.
The cells (20 mg, wet weight) were suspended in 100 µl of an SDS-gel sample buffer [U.K. Laemmli, Nature, 227, 680(1970)] and then heated at 100°C for 10 minutes to extract proteins. The solubilized proteins were fractionated by 12.5% SDS-polyacrylamide gel electrophoresis, and then transferred onto a nitrocellulose filter according to the known Western blotting method [H. Towbin et al., Proc. Natl. Acad. Sci. USA, 76, 4350(1979)]. The screening of the HTLV-I gag protein by a known anti-p19 murine monoclonal antibody GIN 7 [S. Itamura et al., Gene, 38, 57 (1985)] revealed that a gag precursor of 53 kDal was expressed.

Example 5
The transformed yeast cells (50 g wet weight) obtained by cultivating Saccharomyces cerevisiae NA74-3A(p⁻) )/pHTI 511 according to the method of Example 4 were suspended in 50 ml of a 50 mM potassium phosphate buffer (pH 7.4) containing 1.2 M sorbitol and 14 mM 2-mercaptoethanol, and treated with 200 µg/ml of Zymolyase 100T (Seikagaku Kogyo) at room temperature for 1.5 hours. The resulting mixture was diluted with 250 ml of a 50 mM potassium phosphate buffer (pH 7.4) containing 0.1% Triton X-100, 0.1 mM (para-amidinophenyl)methanesulfonyl fluoride (APMSF) and allowed to stand at room temperature for 1.5 hours to disrupt the cells. The lysate was centrifuged at 14,000 rpm for 20 hours to remove the supernatant, and then 125 ml of a 1/150 M phosphate buffer (pH 7.0) containing 8M quanidine hydrochloride was added to the precipitate. The mixture was stirred at room temperature for 2 hours to extract the gag precursor, and then centrifuged at 14,000 rpm for 20 minutes to obtain a supernatant as a crude extract.
The crude extract was diluted fortyfold with a 1/150 M phosphate buffer (pH 7.0) containing 0.5 M urea, 0.25% Nonidet P40, 15 mM EDTA, 1mM phenylmethanesulfonyl fluoride (PMSF) and 0.1 mM APMSF. An anti-p 19 murine monoclonal antibody [Tanaka et al., Gann, 74, 327(1983)]-bound Formyl-Cellulofine (Seikagaku Kogyo) column having a bed volume of 5 ml which was previously equilibrated with the same buffer was charged with the obtained dilution and washed with the above-described buffer for dilution and then a 1/150 M phosphate buffer (pH 7.0) containing 1M quanidine hydrochloride. The HTLV-I gag precursor was eluted with a

6

1/150 M phosphate buffer (pH 7.0) containing 3M quanidine hydrochloride from the column.

The eluate from the antibody column was subjected to high performance liquid chromatography equipped with YMC-PACK C$_4$ (Yamamura Kagaku), and eluted by increasing the concentration of acetonitrile from 20% to 100% linearly in the presence of 0.1% trifluoroacetic acid (TFA). The gag precursor was eluted in an acetonitrile concentration of about 65% later than yeast-derived other proteins. The solvents were removed to yield 117 μg of the gag precursor (the amount is about 1 to 2 %, relative to the total soluble protein).

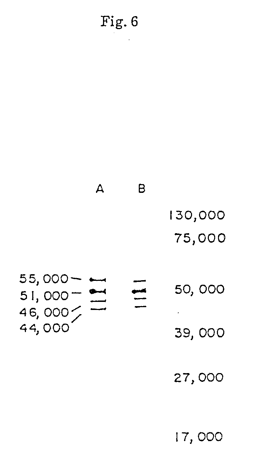

The HTLV-I gag precursor thus obtained was subjected to polyacrylamide gel electrophoresis containing sodium dodecyl sulfate (SDS-PAGE) and stained by the silver-staining method to mainly stain a band of 51 KDal as shown in Fig. 6A. Further, bands of 55, 46 and 44 KDal were observed. This silver-stained image was nearly identical with the image of Western blotting using horseradish peroxidase-conjugated anti-p19 murine monoclonal antibody GIN-7 (Fig. 6B).

**Claims**

1. An expression plasmid for a protein possessing human T cell leukemia virus type I gag antigenicity, characterized in that it contains a DNA that encodes said protein and it is capable of replication in yeasts.

2. The plasmid according to claim 1, wherein the DNA is a DNA whose base sequence is shown in Fig. 3.

3. The plasmid according to claim 1, wherein the plasmid contains a promoter capable of functioning in yeasts upstream from said DNA.

4. The plasmid according to claim 3, wherein the promoter is a promoter selected from the class consisting of GLD promoter, PHO 5 promoter, PGK promoter, ADH promoter, PHO 81 promoter and GAL 10 promoter.

5. The plasmid according to claim 3, wherein the promoter is GLD promoter.

6. The plasmid according to claim 1, wherein the plasmid contains a terminator selected from the class consisting of phosphoglycerate kinase gene terminator, 2μ DNA FLP gene terminator and invertase gene terminator downstream from said DNA.

7. The plasmid according to claim 6, wherein the terminator is phosphoglycerate kinase gene terminator.

8. The plasmid pHTI 501.

9. The plasmid pHTI 511.

10. A yeast transformant containing the plasmid as claimed in claim 1.

11. The transformant according to claim 10, wherein the yeast is a respiratory deficient strain.

12. The transformant according to claim 11, wherein the yeast is Saccharomyces cerevisiae.

13. The transformant Saccharomyces cerevisiae NA74-3A(ρ⁻) /pHTI 501.

14. The transformant Saccharomyces cerevisiae NA74-3A(ρ⁻) /pHTI 511.

15. A method of producing a protein possessing human T cell leukemia virus type I gag antigenicity, which comprises:
cultivating the yeast transformant of claim 10 in a culture medium;
accumulating said protein in said culture medium; and harvesting said protein from said culture medium.

16. The method according to claim 15 wherein the yeast is a respiratory deficient strain.

17. The method according to claim 15 wherein the yeast is Saccharomyces cerevisiae.

18. The method according to claim 15 wherein the transformant is Saccharomyces cerevisiae NA74-3A (ρ⁻)/pHTI 501.

19. The method according to claim 15 wherein the transformant is Saccharomyces cerevisiae NA74-3A (ρ⁻)/pHTI 511.

Fig. 1

Fig. 2

Fig. 3-1

```
  1   Met Gly Gln Ile Phe Ser Arg Ser Ala Ser Pro Ile Pro Arg Pro Pro
 70   ATG GGC CAA ATC TTT TCC CGT AGC GCT AGC CCT ATT CCG CGA CCG CCC

 17   Arg Gly Leu Ala Ala His His Trp Leu Asn Phe Leu Gln Ala Ala Tyr
118   CGG GGG CTG GCC GCT CAT CAC TGG CTT AAC TTC CTC CAG GCG GCA TAT

 33   Arg Leu Glu Pro Gly Pro Ser Ser Tyr Asp Phe His Gln Leu Lys Lys
166   CGC CTA GAA CCC GGT CCC TCC AGT TAC GAT TTC CAC CAG TTA AAA AAA

 49   Phe Leu Lys Ile Ala Leu Glu Thr Pro Ala Arg Ile Cys Pro Ile Asn
214   TTT CTT AAA ATA GCT TTA GAA ACA CCG GCT CGG ATC TGT CCC ATT AAC

 65   Tyr Ser Leu Leu Ala Ser Leu Leu Pro Lys Gly Tyr Pro Gly Arg Val
262   TAC TCC CTC CTA GCC AGC CTA CTC CCA AAA GGA TAC CCC GGC CGG GTG

 81   Asn Glu Ile Leu His Ile Leu Ile Gln Thr Gln Ala Gln Ile Pro Ser
310   AAT GAA ATT TTA CAC ATA CTC ATC CAA ACC CAA GCC CAG ATC CCG TCC

 97   Arg Pro Ala Pro Pro Pro Pro Ser Ser Pro Thr His Asp Pro Pro Asp
358   CGT CCC GCG CCA CCG CCG CCG TCA TCC CCC ACC CAC GAC CCC CCG GAT

113   Ser Asp Pro Gln Ile Pro Pro Pro Tyr Val Glu Pro Thr Ala Pro Gln
406   TCT GAT CCA CAA ATC CCC CCT CCC TAT GTT GAG CCT ACG GCC CCC CAA

129   Val Leu Pro Val Met His Pro His Gly Ala Pro Pro Asn His Arg Pro
454   GTC CTT CCA GTC ATG CAT CCA CAT GGT GCT CCT CCT AAC CAT CGC CCA

145   Trp Gln Met Lys Asp Leu Gln Ala Ile Lys Gln Glu Val Ser Gln Ala
502   TGG CAA ATG AAA GAC CTA CAG GCC ATT AAG CAA GAA GTC TCC CAA GCA
```

EP 0 352 060 A2

Fig. 3-2

```
161   Ala Pro Gly Ser Pro Gln Phe Met Gln Thr Ile Arg Leu Ala Val Gln
550   GCC CCT GGG AGC CCC CAG TTT ATG CAG ACC ATC CGG CTT GCG GTG CAG

177   Gln Phe Asp Pro Thr Ala Lys Asp Leu Gln Asp Leu Leu Gln Tyr Leu
598   CAG TTT GAC CCC ACT GCC AAA GAC CTC CAA GAC CTC CTG CAG TAC CTT

193   Cys Ser Ser Leu Val Ala Ser Leu His His Gln Gln Leu Asp Ser Leu
646   TGC TCC TCC CTC GTG GCT TCC CTC CAT CAC CAG CAG CTA GAT AGC CTT

209   Ile Ser Glu Ala Glu Thr Arg Gly Ile Thr Gly Tyr Asn Pro Leu Ala
694   ATA TCA GAG GCC GAA ACC CGA GGT ATT ACA GGT TAT AAC CCA TTA GCC

225   Gly Pro Leu Arg Val Gln Ala Asn Asn Pro Gln Gln Gln Gly Leu Arg
742   GGT CCC CTC CGT GTC CAA GCC AAC AAT CCA CAA CAA CAA GGA TTA AGG

241   Arg Glu Tyr Gln Gln Leu Trp Leu Ala Ala Phe Ala Ala Leu Pro Gly
790   CGA GAA TAC CAG CAA CTC TGG CTC GCC GCC TTC GCC GCC CTG CCG GGG

257   Ser Ala Lys Asp Pro Ser Trp Ala Ser Ile Leu Gln Gly Leu Glu Glu
838   AGT GCC AAA GAC CCT TCC TGG GCC TCT ATC CTC CAA GGC CTG GAG GAG

273   Pro Tyr His Ala Phe Val Glu Arg Leu Asn Ile Ala Leu Asp Asn Gly
886   CCT TAC CAC GCC TTC GTA GAA CGC CTC AAC ATA GCT CTT GAC AAT GGG

289   Leu Pro Glu Gly Thr Pro Lys Asp Pro Ile Leu Arg Ser Leu Ala Tyr
934   CTG CCA GAA GGC ACG CCC AAA GAC CCC ATC TTA CGT TCC TTA GCC TAC

305   Ser Asn Ala Asn Lys Glu Cys Gln Lys Leu Leu Gln Ala Arg Gly His
982   TCC AAT GCA AAC AAA GAA TGC CAA AAA TTA CTA CAG GCC CGA GGA CAC
```

EP 0 352 060 A2

Fig. 3-3

321    Thr Asn Ser Pro Leu Gly Asp Met Leu Arg Ala Cys Gln Thr Trp Thr
1030  ACT AAT AGC CCT CTA GGA GAT ATG TTG CGG GCT TGT CAG ACC TGG ACC

337    Pro Lys Asp Lys Thr Lys Val Leu Val Val Gln Pro Lys Lys Pro Pro
1078  CCC AAA GAC AAA ACC AAA GTG TTA GTT GTC CAG CCT AAA AAA CCC CCC

353    Pro Asn Gln Pro Cys Phe Arg Cys Gly Lys Ala Gly His Trp Ser Arg
1126  CCA AAT CAG CCG TGC TTC CGG TGC GGG AAA GCA GGC CAC TGG AGT CGG

369    Asp Cys Thr Gln Pro Arg Pro Pro Pro Gly Pro Cys Pro Leu Cys Gln
1174  GAC TGC ACT CAG CCT CGT CCC CCC CCC GGG CCA TGC CCC CTA TGT CAA

385    Asp Pro Thr His Trp Lys Arg Asp Cys Pro Arg Leu Lys Pro Thr Ile
1222  GAC CCA ACT CAC TGG AAG CGA GAC TGC CCC CGC CTA AAG CCC ACT ATC

401    Pro Glu Pro Glu Pro Glu Glu Asp Ala Leu Leu Leu Asp Leu Pro Ala
1270  CCA GAA CCA GAG CCA GAG GAA GAT GCC CTC CTA TTA GAC CTC CCC GCT

417    Asp Ile Pro His Pro Lys Asn Ser Ile Gly Gly Glu Val ***
1318  GAC ATT CCA CAC CCA AAA AAC TCC ATA GGG GGG GAG GTT TAA

Fig. 4

EP 0 352 060 A2

Fig. 5

Fig. 6